# EUROPEAN PATENT SPECIFICATION

(11) **EP 3 155 974 B1**
(45) Date of publication and mention of the grant of the patent: **25.12.2019**
(21) Application number: 15809134.8
(22) Date of filing: 28.04.2015
(51) Int. Cl.: A61B 17/00, A61F 2/24, A61L 27/04, A61L 27/34

(54) **CERCLAGE ROPE FOR MITRAL VALVE CERCLAGE PROCEDURE HAVING ARCHED CORONARY ARTERY PROTECTOR INTEGRALLY FORMED THEREWITH**
CERCLAGESEIL FÜR MITRALKLAPPEN-CERCLAGEEINGRIFF MIT INTEGRAL DARIN GEFORMTEM, GEWÖLBTEM HERZARTERIENPROTEKTOR
CORDON DE CERCLAGE POUR PROCÉDURE DE CERCLAGE DE VALVE MITRALE AYANT UN PROTECTEUR D'ARTÈRE CORONAIRE ARQUÉ FORMÉ DE FAÇON INTÉGRÉE À CELUI-CI

(30) Priority: 16.06.2014 KR 20140072525
(43) Date of publication of application: 19.04.2017
(73) Proprietor: Taupnumedical Co. Ltd., Busan 609-836 (KR)
(72) Inventor: KIM, June-hong, Busan 608-776 (KR)
(74) Representative: HGF Europe LLP
(86) International application number: PCT/KR2015/004205
(87) International publication number: WO 2015/194754

(56) References cited:
- WO-A2-2008/089044
- JP-A- 2003 521 261
- JP-A- 2012 157 378
- US-A1- 2010 049 314
- US-A1- 2011 054 597

## Description

### Technical Field

The present invention relates to a cerclage rope for a mitral valve cerclage procedure having an arched coronary artery protector integrally formed therewith and, more particularly, a cerclage rope for a mitral valve cerclage procedure having an arched coronary artery protector integrally formed therewith for protecting coronary arteries in a mitral valve cerclage procedure.

### Background Art

The heart is the center of the human circulatory system that pumps blood through our body. It is a muscle that pumps the blood only in one direction. In order for the heart to effectively maintain this unidirectional flow of blood, it must have properly functioning valves that prevent back flow through its system, or regurgitation. The heart is divided into four chambers: right and left atrium, and right and left ventricles. The four chambers are connected to the aorta, inferior and superior vena cava, pulmonary artery, and pulmonary veins.

The mitral valve (MV) separates left atrium from left ventricle while the tricuspid valve (TV) separates right atrium from the right ventricle. The aortic valve (AV) is located between the left ventricle and the aorta while the pulmonary valve (PV) is located between the right ventricle and the pulmonary artery.

Generally, valves should open and close completely with every heart beat or contraction. Incomplete opening or closing of the valves causes improper flow of blood. Valvular diseases are divided into two categories, regurgitation and stenosis. Regurgitation is a failure of a valve to close completely and stenosis is a failure of a valve to open completely.

Mitral valve regurgitation (MVR) is a common cardiac valve disorder that is caused by incomplete closure of the mitral valve (MV). The MV is located between the left atrium and the left ventricle. Over time, MVR places a burden on the heart and worsens its ability to pump blood properly. Such stress on the heart will ultimately lead to heart failure.

Traditional treatment for worsening MVR requires open heart surgery with sternotomy or thoracotomy with cardiac arrest and cardio-pulmonary bypass. Once the chest is open and access to heart is gained, the MV is either repaired or replaced using an artificial valve. Although very effective, this open-heart procedure is a high risk surgery accompanied by substantial morbidity and prolonged convalescence. Mortality due to surgery itself can be as high as 5%. As a result, the procedure often is not offered to patients who are insufficiently symptomatic to justify the surgical risk and morbidity, or to patients with substantial co-morbidity. It is reserved only for those with severe symptomatic MVR.

This high morbidity rate of open heart surgery has motivated further research into developing a safer and less risky alternative to repair MV. Much of the research involves use of cardiac catheterization. Recently, the inventor of the present invention presented a thesis regarding "mitral valve cerclage coronary sinus annuloplasty (MVA)" showing outstanding results of MVR treatment through applying circular pressure around the mitral annulus (MA). This thesis has been filed through PCT as an international patent application (PCT application number PCT/US2007/023876) and has been published (International publication number WO2008/060553).

The aforementioned thesis and published patent application disclosed the mitral cerclage coronary annuloplasty (MVA) procedure. Briefly explained, a catheter is placed in coronary sinus after accessing the right atrium through the jugular vein, and then a suture (referred to as a cerclage suture) is passed through the proximal septal vein. This cerclage suture can easily pass through right ventricular outflow tract (RVOT), and this inventor defines this technique as "simple mitral cerclage annuloplasty". Then, the cerclage suture can be easily pulled into the right atrium thus placing the cerclage suture circumferentially around the mitral annulus. Once positioned, tension is applied to the cerclage suture, thereby tightening the mitral valve. This brings together the two lobes of the MV so that they are approximated and reduce the size of its incomplete closure. This procedure can theoretically obtain very similar results to those that conventional surgeries can obtain by directly tightening the mitral annulus, and shows an immediate reduction of MVR.

However, the international patent application has problems to be solved. First, there is a need for a tension locking device that can apply and maintain appropriate tension in a cerclage suture at the site; and second, the tension is maintained by a relatively very thin cerclage suture (0.014inch nylon was used in the research of the related art and this thickness can be change), so adjacent tissues may be injured.

In order to solve these problems, the inventor(s) has proposed a "Device for delivering optimal tension safely and effectively in cerclage annuloplasty procedure" in Korean Patent Application No. 2009-0080708 (filed on 28 August, 2009). (Korean Patent No. 10-1116867, registered on 8 February, 2012)

The Patent Document proposed that a cerclage suture be covered with a CS tube and a TV tube in order not to injure tissues (that is, protect tissues with a tissue protector) and a knot at an end of the cerclage suture is delivered to the top of the tissue protector using a knot deliverer, thereby completing a cerclage procedure.

In this Patent Document, nylon was used as the cerclage suture passing through the tissue protector (CSTV TUBE). Nylon is a biocompatible material, is not deformed under even predetermined tension, and has high durability, so it is suitable as a cerclage suture for a cerclage procedure.

However, the cerclage nylon suture is not shown in a fluoroscopic image (X-ray) that is used as a guided image in a mitral valve cerclage procedure, so the mitral valve cerclage procedure is difficult to perform. Further, it is required to put the cerclage nylon suture inside through a catheter having a small diameter in order to move the cerclage nylon suture to a target portion in a heard in a mitral valve cerclage procedure, but pushability is low and it is difficult to delivery the suture due to the characteristics of nylon.

Further, the arched portion that is a protector for a coronary sinus proposed in the international patent application (PCT application number: PCT/US2007/023876) is not tightly fit in a cerclage suture, so it can be easily moved by the motion of a heart, and accordingly, safety cannot be secured.

US 2011/0054597 A1 discloses a mitral cerclage annuloplasty apparatus where a suture is fed through a coronary sinus and tricuspid valve protective device starting at a coronary sinus extension of one tube of a double-tubed stem portion, a coronary sinus tube, an arched coronary protective device, a tricuspid valve tube, and back out through a tricuspid valve extension of the other tube of said double-tubed stem portion, or in reverse direction.

### Disclosure

### Technical Problem

The present invention has been made in an effort to solve the problems and an object of the present invention is to provide a cerclage rope for a mitral valve cerclage procedure having an arched coronary artery protector integrally formed therewith. The cerclage rope is not deformed even by predetermined tension, has high durability, can be seen by an X-ray, whereby it is possible to visually recognize its position and whether it is moved. Further, the cerclage rope makes a cerclage procedure easy due to high pushability, does not require a process of separately inserting only an arched coronary artery protector, and prevents the arched coronary artery protector from easily moving even by the pulsation of a heart.

However, the objects of the present invention are not limited to those stated above and other objects not stated above may be clear to those skilled in the art from the following description.

### Technical Solution

In order to achieve the objects of the present invention, a cerclage rope for a mitral valve cerclage procedure having arched coronary artery protector integrally formed therewith according to an embodiment of the present invention, includes: a cerclage rope having metal wires and coated with biocompatible synthetic resin; an arched coronary artery protector receiving the cerclage rope and protecting a coronary sinus; and a coating integrating the arched coronary artery protector and the cerclage rope by partially or fully covering the coronary artery protector and by partially covering the cerclage rope.

A metal may be stainless steel and the synthetic resin may be nylon.

The wires may be each formed by twisting a plurality of thin wires, and preferably the wires may be each formed by twisting a plurality of thin wires into a strand and then twisting a plurality of the strands.

The coating may be Teflon and the Teflon may be formed by covering the arched coronary artery protector and the cerclage rope with a Teflon sheet (PTFE sheet) and then thermally pressing the Teflon sheet.

### Advantageous Effects

As described above, the cerclage rope for a metal valve cerclage procedure of the present invention is made of metal and biocompatible synthetic resin, so it is suitable for a living body, is not easily deformed even by predetermined tension, has high durability, and can be seen by an X-ray, whereby the position of the cerclage rope and whether the it is moved forward can be visually checked. Further, the cerclage rope is useful for a mitral valve cerclage procedure due to its high pushability.

Further, according to the present invention, since the arched coronary artery protector that is a coronary sinus protector is integrated with a cerclage rope, there is no need for a process of separately inserting only an arched coronary artery protector into a cerclage rope in a cerclage procedure and the arched coronary protector is not easily moved even by the pulsation of a heart, so safety in a cerclage procedure can be increased.

### Description of Drawings

FIG. 1 is a perspective view of a cerclage rope for a mitral valve cerclage procedure having arched coronary artery protector integrally formed therewith and a tissue protector according to an embodiment of the present invention.
FIG. 2 is a partial cross-sectional view of the cerclage rope for a mitral valve cerclage procedure having arched coronary artery protector integrally formed therewith according to an embodiment of the present invention.
FIG. 3 is a cross-sectional view of the cerclage rope for a mitral valve cerclage procedure having arched coronary artery protector integrally formed therewith according to an embodiment of the present invention.
FIG. 4 is a schematic view showing a mitral valve cerclage procedure using the cerclage rope for a mitral valve cerclage procedure having arched coronary artery protector integrally formed therewith according to an embodiment of the present invention.

### Mode for Invention

Hereinafter, a cerclage rope for a mitral valve cerclage procedure having arched coronary artery protector integrally formed therewith according to an embodiment of the present invention is described in detail.

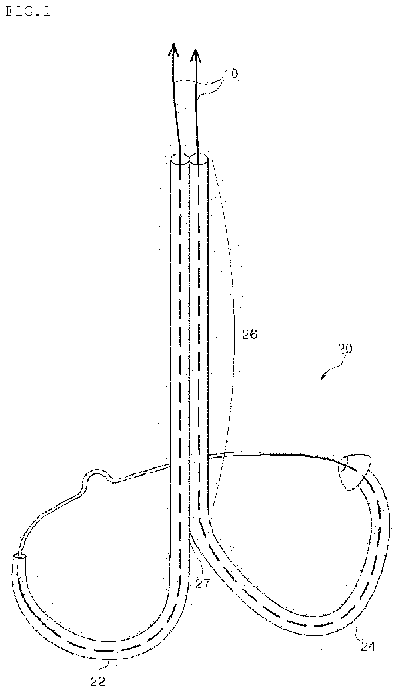

FIG. 1 is a perspective view of a cerclage rope for a mitral valve cerclage procedure having arched coronary artery protector integrally formed therewith and a tissue protector according to an embodiment of the present invention.

Referring to FIG. 1, a cerclage rope is designed to be inserted into a tissue protector 20 for protecting tissues in a body to tie the mitral annulus, so it is an important element for curing mitral valve regurgitation in a mitral valve cerclage procedure.

In general, parts having a possibility of being damaged in mitral cerclage coronary sinus annuloplasty (MVA) are the coronary sinus (CS), the tricuspid valve (TV), and ventricular septum (IVS), so the tissue protector 20 covers the cerclage rope 10 to protect the tissues therearound.

The tissue protector 20 includes: a hollow coronary sinus tube (CS tube) 22 for protecting coronary sinus tissues; a hollow tricuspid valve tube (TV tube) 24 for protecting the tissues of the tricuspid valve and the ventricular septum; and a stem 26 composed of a coronary sinus tube and a tricuspid valve tube attached to each other. In other words, the tissue protector of the present invention is composed of a hollow coronary sinus tube (CS tube) for protecting coronary sinus tissues and a hollow tricuspid valve tube (TV tube) for protecting the tissues of the tricuspid valve and the ventricular septum, in which the coronary sinus tube and the tricuspid valve tube are attached to each other from the top to a predetermined portion and are separated at the lower portions. The coronary sinus tube 22 protects coronary sinus tube tissues against a cerclage rope inserted into the coronary sinus and the tricuspid valve tube 24 protects the tissues of the tricuspid valve and the ventricular septum against a cerclage tube inserted into the tricuspid valve.

The coronary sinus tube (CS tube) and the tricuspid valve tube (TV tube) may be catheters made of rubber or synthetic resin or metallic coil springs. Coil springs are soft and flexible, can move with the pulsation of a heart, and can flexibly return after changing the shapes. Stainless steel may be used for the coil springs.

The cerclage rope 10 is named as such because it has a piece of string that comes out of a body after going around the coronary sinus (CS), the tricuspid valve (TV), and the ventricular septum in mitral cerclage coronary sinus annuloplasty (MVA), and when it comes out of a body, it is divided into two pieces at one end and the other end. That is, as shown in FIG. 1, it is one continuous rope making a circle. The cerclage rope is named as such because it has rope shape and is also usually called a "cerclage suture".

The mitral valve cerclage procedure having arched coronary artery protector integrally formed therewith according to an embodiment of the present invention includes a cerclage rope 10, an arched coronary artery protector 30, and a coating 40.

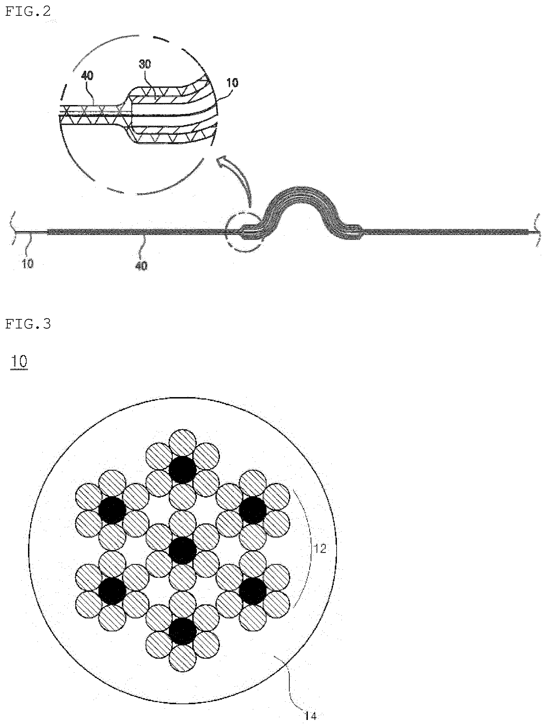

FIG. 3 is a cross-sectional view of the cerclage rope for a mitral valve cerclage procedure having arched coronary artery protector integrally formed therewith according to an embodiment of the present invention.

Referring to FIG. 3, the cerclage rope 10 of the present invention includes metallic wires 12 therein and is covered with a nylon coating 14 on the outer side. That is, for the cerclage rope 10, wires 12 made of metal, particularly, noncorrosive stainless steel that does not corrode even if it comes in contact with a human body due to partial loss of the coating are used so that an operator can check the progress using X-rays during mitral valve cerclage procedure, and they are covered with the nylon coating 14 made of biocompatible synthetic resin.

The nylon cerclage suture is replaced by the cerclage rope of the present invention in a mitral valve cerclage procedure.

The wires 12 in the cerclage rope 10 are made of metal, preferably, stainless steel that does not corrode even if it comes in contact with a human body. For example, AISI304 may be used, but it is not limited thereto.

The wires 12 may be single wires, but preferably, formed by twisting a plurality of thin wires. The cerclage wires 12 shown in FIG. 3 are based on the most preferable embodiment and are each formed by making strands with seven pieces of stainless wire each having a diameter of 0.035 ∼ 0.06mm and then twisting the seven strands.

A cerclage procedure requires tensile force (breaking strength) of 100N or more and tensile strength (T/S) of 2250N/m² or more of a cerclage rope. The cerclage rope formed by twisting a total of 49 (7x7) pieces of thin stainless steel wires secures the tensile force and tensile strength. That is, the cerclage rope not a single wire, but formed by twisting a plurality of wires that have high tensile force and tensile strength, high flexibility, and high pushability.

The coating 14 is a part covering the wire 12 and is made of biocompatible synthetic resin or preferably nylon. The outer diameter D of the entire cerclage rope including the coasting may be 0.5mm or less, the coating should be smooth, and there has to be no burned traces. This is because the cerclage rope is supposed to the inserted into a human body through a catheter in a cerclage procedure.

The nylon coating of the wire 12 is formed by common extrusion molding. That is, a gel obtained by melting solid nylon is applied to a wire that is continuously supplied from an extruder and is then cooled by water, thereby forming the rope.

As described above, the cerclage rope of the present invention includes stainless steel wires therein, so it is possible to visually check the position of the cerclage rope and whether the cerclage rope is moved during a cerclage procedure. Further, the stainless steel wires make a cerclage procedure easy due to the high flexibility and pushability thereof, and they are not harmful to a human body because they are coated with biocompatible nylon, which has high durability.

The arched coronary artery protector 30 of the present invention is a part that protects coronary arteries. In a cerclage procedure, a cerclage rope is inserted into the coronary sinus, but when the cerclage is tightened to block the mitral annulus of a patient with mitral valve regurgitation, the cerclage rope moving on a coronary artery in the coronary sinus presses the coronary artery, so the flow of blood in the coronary artery is blocked and the coronary artery is injured. Accordingly, a technology of protecting a coronary artery by inserting an arch into a coronary sinus with a coronary artery to be protected has been disclosed in International Patent Application Publication (WO2008/060553, published on 22 May, 2008). The arched coronary artery protector 30 is called a "coronary sinus protector" because of it being inserted into a coronary sinus, or is called a "coronary artery protector" because it protects coronary arteries. The name "arched coronary artery protector" is given because the coronary sinus protector has an arc shape to protect coronary arteries when being inserted in the coronary sinus.

The arched coronary artery protector of the present invention may be made of synthetic resin or metal, preferably, stainless steel.

The present invention is characterized in that the 'arched coronary artery protector' is a coronary sinus protector integrally fixed to a cerclage rope.

In the present invention, the coating 40 is a part integrating the arched coronary artery protector 30 and the cerclage rope 10 by partially covering the arched coronary artery protector 30 and the cerclage rope 10.

In a cerclage procedure, the portion, which is not protected by a tissue protector (CSTV tube), of the cerclage rope is relatively vulnerable to erosion, but the portion is covered with a coating in the cerclage rope of the present invention, so safety can be improved.

If the entire cerclage rope is made thick to prevent erosion of the cerclage rope, the ventricular septum may be severely injured because a large catheter is required and the thick rope passes through the ventricular septum due to the characteristics of a cerclage procedure. However, since a cerclage rope is partially covered with a coating in the present invention, this problem does not occur.

The coating is made of a biocompatible material. The biocompatible material may be Teflon (ePTFE, expanded polytetrafluoroethylene).

Teflon can be coated on the rope in various ways. For example, the arched coronary artery protector and the cerclage rope is covered with a Teflon sheet (ePTFE sheet) and then the Teflon is fixed by thermal pressing.

When the arched coronary artery protector is integrally fixed to the cerclage rope, as described above, it is possible to remove the process of separately inserting an arched coronary artery protector through a cerclage rope during a cerclage procedure. Further, since the arched coronary artery protector is fixed to the cerclage rope, the arched coronary artery protector is not independently moved even by the pulsation of the heart, so safety of the cerclage procedure can be increased.

A process of performing a mitral valve cerclage procedure using the cerclage rope of the present invention is described hereafter.

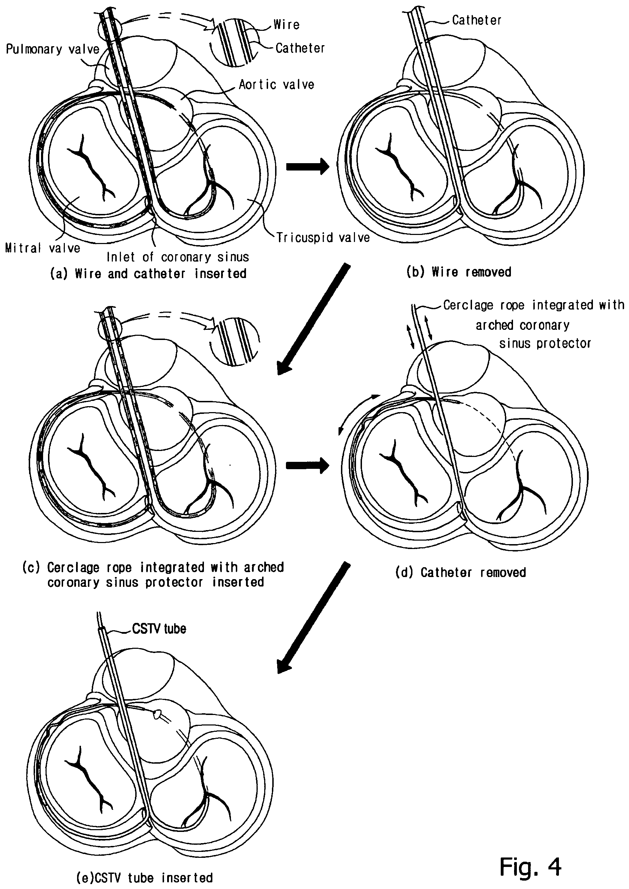

FIG. 4 is a schematic view showing a mitral valve cerclage procedure using the cerclage rope for a mitral valve cerclage procedure having arched coronary artery protector integrally formed therewith according to an embodiment of the present invention.

Referring to FIG. 4, a surgeon inserts a wire into a catheter and then gradually pushes the catheter with the wire into a desired portion in a tissue of a human body (a). The wire has pushability, so the catheter is gradually inserted with the wire into a desired portion in a tissue of a human body in a way of inserting a thin wire and then inserting the catheter, that is, in a way where the wire goes first and the catheter follows the wire. The wire is a common wire (RTCA wire) that is currently used for intervention and the material of the catheter is soft rubber or synthetic resin.

Next, the surgeon takes out the wire from the human body. Then, only the catheter is left (b).

Next, the surgeon inserts the cerclage rope integrated with an arched coronary artery protector into the catheter (c). Since the cerclage rope integrated with an arched coronary artery protector has high flexibility and pushability, it is smoothly inserted and circulated through the catheter.

The surgeon checks using imaging system outside the patient whether the arched coronary artery protector can protect a coronary artery from the cerclage rope at an accurate position, that is, over the coronary artery. That is, the surgeon finds out the accurate position and places the arched coronary artery protector to the position by repeatedly pushing and pulling both ends of the cerclage rope, as indicated by an arrow.

The operator takes out the catheter from the human body when the cerclage rope is fully inserted (d).

Finally, the operator inserts a tissue protector (CSTV tube) (e), thereby completing the mitral valve cerclage procedure.

An important matter in this process is that since the arched coronary artery protector is integrally fixed to the cerclage rope, there is no need for the process of inserting an arched coronary artery protector through a cerclage rope.

A case when the tissue protector (CSTV tube) designed by the inventor (s) is used in the process of performing a mitral valve cerclage procedure was described above, but the tissue protector (CSTV tube) is not necessary and the cerclage rope of the present invention is coated with biocompatible nylon and has high flexibility and pushability, so it can be independently used without a tissue protector (CSTV tube).

As described above, the cerclage rope integrated with an arched coronary artery protector of the present invention plays a very important role in a mitral valve cerclage procedure and is considered as a core element that determines success and failure of a cerclage procedure.

### Industrial Applicability

The present invention can be applied to the field of a cerclage rope for a mitral valve cerclage procedure having arched coronary artery protector integrally formed therewith and, more particularly, the field of a cerclage rope for a mitral valve cerclage procedure having arched coronary artery protector integrally formed therewith for protecting coronary arteries in a mitral valve cerclage procedure.

## Claims

1. A cerclage rope for a mitral valve cerclage procedure having arched coronary artery protector integrally formed therewith, the cerclage rope comprising:
a cerclage rope (10) having metal wires (12) therein and a coating (14) made of biocompatible synthetic resin and covering the wires;
an arched coronary artery protector (30) receiving the cerclage rope and protecting a coronary sinus; and
a coating (40) integrating the arched coronary artery protector (30) and the cerclage rope (10) by partially or fully covering the coronary artery protector (30) and by partially covering the cerclage rope (10).

2. The cerclage rope of claim 1, wherein the wires are each formed by twisting a plurality of thin wires.

3. The cerclage rope of claim 1, wherein the wires are each formed by twisting a plurality of thin wires into a strand and then twisting a plurality of the strands.

4. The cerclage rope of claim 1, wherein the coating (40) is made of a biocompatible material.

5. The cerclage rope of claim 1, wherein the coating (40) is Teflon.

6. The cerclage rope of claim 5, wherein the Teflon is formed by covering the arched coronary artery protector (30) and the cerclage rope (10) with a Teflon sheet (PTFE sheet) and then thermally pressing the Teflon sheet.

7. The cerclage rope of claim 1, wherein the metal is stainless steel and the synthetic resin is nylon.

## Patentansprüche

1. Cerclageseil für einen Mitralklappen-Cerclageeingriff mit integral darin gebildetem, gewölbtem Koronararterienprotektor, wobei das Cerclageseil umfasst:
ein Cerclageseil (10), das Metalldrähte (12) darin und eine Beschichtung (14) aufweist, die aus einem biokompatiblen Kunstharz hergestellt ist und die Drähte bedeckt;
einen gewölbten Koronararterienprotektor (30), der das Cerclageseil aufnimmt und einen Koronarsinus schützt; und
eine Beschichtung (40), die den gewölbten Koronararterienprotektor (30) und das Cerclageseil (10) durch partielles oder vollständiges Bedecken des Koronararterienprotektors (30) und durch partielles Bedecken des Cerclageseils (10) einbindet.

2. Cerclageseil nach Anspruch 1, wobei die Drähte jeweils durch Verdrillen einer Vielzahl von dünnen Drähten gebildet werden.

3. Cerclageseil nach Anspruch 1, wobei die Drähte jeweils durch Verdrillen einer Vielzahl von dünnen Drähten in eine Litze und dann Verdrillen einer Vielzahl von den Litzen gebildet werden.

4. Cerclageseil nach Anspruch 1, wobei die Beschichtung (40) aus einem biokompatiblen Material hergestellt ist.

5. Cerclageseil nach Anspruch 1, wobei die Beschichtung (40) Teflon ist.

6. Cerclageseil nach Anspruch 5, wobei das Teflon durch Bedecken des gewölbten Koronararterienprotektors (30) und des Cerclageseils (10) mit einer Teflonfolie (PTFE-Folie) und dann Wärmepressen der Teflonfolie gebildet wird.

7. Cerclageseil nach Anspruch 1, wobei das Metall Edelstahl ist und das Kunstharz Nylon ist.

## Revendications

1. Cordon de cerclage pour une procédure de cerclage de valve mitrale ayant un protecteur d'artère coronaire arqué formé de façon intégrée à celui-ci, le cordon de cerclage comportant :
un cordon de cerclage (10) ayant des fils métalliques (12) en son sein et un revêtement (14) fait d'une résine synthétique biocompatible et couvrant les fils ;
un protecteur d'artère coronaire arqué (30) recevant le cordon de cerclage et protégeant un sinus coronaire ; et
un revêtement (40) intégrant le protecteur d'artère coronaire arqué (30) et le cordon de cerclage (10) par recouvrement partiel ou total du protecteur d'artère coronaire (30) et par recouvrement partiel du cordon de cerclage (10).

2. Cordon de cerclage selon la revendication 1, dans lequel les fils sont chacun constitués par torsion d'une pluralité de fils minces.

3. Cordon de cerclage selon la revendication 1, dans lequel les fils sont chacun constitués par torsion d'une pluralité de fils minces en un brin, puis par torsion d'une pluralité des brins.

4. Cordon de cerclage selon la revendication 1, dans lequel le revêtement (40) est fait d'un matériau biocompatible.

5. Cordon de cerclage selon la revendication 1, dans lequel le revêtement (40) est du Téflon.

6. Cordon de cerclage selon la revendication 5, dans lequel le Téflon est constitué par recouvrement du protecteur d'artère coronaire arqué (30) et du cordon de cerclage (10) avec une feuille de Téflon (feuille PTFE), puis par pressage thermique de la feuille de Téflon.

7. Cordon de cerclage selon la revendication 1, dans lequel le métal est de l'acier inoxydable et la résine synthétique est du nylon.
